# EUROPEAN PATENT APPLICATION

(11) **EP 3 578 671 A1**
(43) Date of publication of application: **11.12.2019**
(21) Application number: 18382393.9
(22) Date of filing: 05.06.2018
(51) Int. Cl.: C12Q 1/6883

(54) **METHOD FOR EVALUATING RISK OF CARDIOVASCULAR DISEASE**

(71) Applicant: Universitat Autònoma de Barcelona, 08193 Bellaterra (Barcelona) (ES); Gendiag, S.L., 08950 Esplugues de Llobregat (Barcelona) (ES)
(72) Inventor: Aluja Paris, Maria Pilar, 08193 Bellaterra (Cerdanyola del Vallès) (ES); Pereira Dos Santos, Cristina Maria, 08193 Bellaterra (Cerdanyola del Vallès) (ES); Umbria Vivancos, Miriam, 08940 Cornellà de Llobregat (ES); Castillo Fernandez, Sergio, 08950 Esplugues de Llobregat (Barcelona) (ES); Ortega Serrano, Israel, 08950 Esplugues de Llobregat (Barcelona) (ES); Salas Perez-Rasilla, Eduardo, 08950 Esplugues de Llobregat (Barcelona) (ES)

(57) **Abstract**

Invention relates to *in vitro* methods for evaluating the risk of suffering from a cardiovascular disease, in which it is detected if one or more genetic variants are present in D-loop sequence of mitochondrial DNA. The invention also relates to particular genetic variations (SNPs) in this mitochondrial DNA.

SEQUENCE LISTING

<110>  UNIVERSITAT AUTÒNOMA DE BARCELONA

<120>  Method for evaluating risk of cardiovascular disease

<130>  T2017042

<160>  9

<170>  PatentIn version 3.5

<210>  1
<211>  20
<212>  DNA
<213>  Artificial

<220>
<223>  forward primer

<400>  1
atacaccagt cttgtaaacc                                                    20


<210>  2
<211>  20
<212>  DNA
<213>  Artificial

<220>
<223>  reverse primer

<400>  2
ttgaggaggt aagctacata                                                    20


<210>  3
<211>  20
<212>  DNA
<213>  Artificial

<220>
<223>  forward primer

<400>  3
gaactgtatc cgacatctgg                                                    20


<210>  4
<211>  20
<212>  DNA
<213>  Artificial

<220>
<223>  reverse primer

<400>  4
ggttaatagg gtgatagacc                                                    20


<210>  5
<211>  20
<212>  DNA

<213>  Artificial

<220>
<223>  reverse primer

<400>  5
ggggtttggt ggaaattttt                                                    20


<210>  6
<211>  16569
<212>  DNA
<213>  Homo sapiens


<220>
<221>  misc_feature
<222>  (3107)..(3107)
<223>  n is a, c, g, or t

<400>  6
gatcacaggt ctatcaccct attaaccact cacgggagct ctccatgcat ttggtatttt      60

cgtctggggg gtatgcacgc gatagcattg cgagacgctg gagccggagc accctatgtc     120

gcagtatctg tctttgattc ctgcctcatc ctattattta tcgcacctac gttcaatatt     180

acaggcgaac atacttacta aagtgtgtta attaattaat gcttgtagga cataataata     240

acaattgaat gtctgcacag ccactttcca cacagacatc ataacaaaaa atttccacca     300

aaccccccct cccccgcttc tggccacagc acttaaacac atctctgcca aaccccaaaa     360

acaaagaacc ctaacaccag cctaaccaga tttcaaattt tatcttttgg cggtatgcac     420

ttttaacagt caccccccaa ctaacacatt attttcccct cccactccca tactactaat     480

ctcatcaata caacccccgc ccatcctacc cagcacacac acaccgctgc taaccccata     540

ccccgaacca accaaacccc aaagacaccc cccacagttt atgtagctta cctcctcaaa     600

gcaatacact gaaaatgttt agacgggctc acatcacccc ataaacaaat aggtttggtc     660

ctagcctttc tattagctct tagtaagatt acacatgcaa gcatccccgt tccagtgagt     720

tcaccctcta aatcaccacg atcaaaagga acaagcatca agcacgcagc aatgcagctc     780

aaaacgctta gcctagccac acccccacgg gaaacagcag tgattaacct ttagcaataa     840

acgaaagttt aactaagcta tactaacccc agggttggtc aatttcgtgc cagccaccgc     900

ggtcacacga ttaacccaag tcaatagaag ccggcgtaaa gagtgtttta gatcaccccc     960

tccccaataa agctaaaact cacctgagtt gtaaaaaact ccagttgaca caaaatagac    1020

tacgaaagtg gctttaacat atctgaacac acaatagcta agacccaaac tgggattaga    1080

taccccacta tgcttagccc taaacctcaa cagttaaatc aacaaaactg ctcgccagaa    1140

cactacgagc cacagcttaa aactcaaagg acctggcggt gcttcatatc cctctagagg    1200

agcctgttct gtaatcgata aaccccgatc aacctcacca cctcttgctc agcctatata    1260

ccgccatctt cagcaaaccc tgatgaaggc tacaaagtaa gcgcaagtac ccacgtaaag 1320

acgttaggtc aaggtgtagc ccatgaggtg gcaagaaatg ggctacattt tctaccccag 1380

aaaactacga tagcccttat gaaacttaag ggtcgaaggt ggatttagca gtaaactaag 1440

agtagagtgc ttagttgaac agggccctga agcgcgtaca caccgcccgt caccctcctc 1500

aagtatactt caaaggacat ttaactaaaa cccctacgca tttatataga ggagacaagt 1560

cgtaacatgg taagtgtact ggaaagtgca cttggacgaa ccagagtgta gcttaacaca 1620

aagcacccaa cttacactta ggagatttca acttaacttg accgctctga gctaaaccta 1680

gccccaaacc cactccacct tactaccaga caaccttagc caaaccattt acccaaataa 1740

agtataggcg atagaaattg aaacctggcg caatagatat agtaccgcaa gggaaagatg 1800

aaaaattata accaagcata atatagcaag gactaacccc tataccttct gcataatgaa 1860

ttaactagaa ataactttgc aaggagagcc aaagctaaga cccccgaaac cagacgagct 1920

acctaagaac agctaaaaga gcacacccgt ctatgtagca aaatagtggg aagatttata 1980

ggtagaggcg acaaacctac cgagcctggt gatagctggt tgtccaagat agaatcttag 2040

ttcaacttta aatttgccca cagaaccctc taaatcccct tgtaaattta actgttagtc 2100

caaagaggaa cagctctttg gacactagga aaaaaccttg tagagagagt aaaaaattta 2160

acacccatag taggcctaaa agcagccacc aattaagaaa gcgttcaagc tcaacaccca 2220

ctacctaaaa aatcccaaac atataactga actcctcaca cccaattgga ccaatctatc 2280

accctataga agaactaatg ttagtataag taacatgaaa acattctcct ccgcataagc 2340

ctgcgtcaga ttaaaacact gaactgacaa ttaacagccc aatatctaca atcaaccaac 2400

aagtcattat taccctcact gtcaacccaa cacaggcatg ctcataagga aaggttaaaa 2460

aaagtaaaag gaactcggca aatcttaccc cgcctgttta ccaaaaacat cacctctagc 2520

atcaccagta ttagaggcac cgcctgccca gtgacacatg tttaacggcc gcggtaccct 2580

aaccgtgcaa aggtagcata atcacttgtt ccttaaatag ggacctgtat gaatggctcc 2640

acgagggttc agctgtctct tacttttaac cagtgaaatt gacctgcccg tgaagaggcg 2700

ggcataacac agcaagacga gaagacccta tggagcttta atttattaat gcaaacagta 2760

cctaacaaac ccacaggtcc taaactacca aacctgcatt aaaaatttcg gttggggcga 2820

cctcggagca gaacccaacc tccgagcagt acatgctaag acttcaccag tcaaagcgaa 2880

ctactatact caattgatcc aataacttga ccaacggaac aagttaccct agggataaca 2940

gcgcaatcct attctagagt ccatatcaac aatagggttt acgacctcga tgttggatca 3000

ggacatcccg atggtgcagc cgctattaaa ggttcgtttg ttcaacgatt aaagtcctac 3060

gtgatctgag ttcagaccgg agtaatccag gtcggtttct atctacnttc aaattcctcc 3120

ctgtacgaaa ggacaagaga aataaggcct acttcacaaa gcgccttccc ccgtaaatga 3180

tatcatctca acttagtatt atacccacac ccacccaaga acagggtttg ttaagatggc 3240

agagcccggt aatcgcataa aacttaaaac tttacagtca gaggttcaat tcctcttctt 3300

aacaacatac ccatggccaa cctcctactc ctcattgtac ccattctaat cgcaatggca 3360

ttcctaatgc ttaccgaacg aaaaattcta ggctatatac aactacgcaa aggccccaac 3420

gttgtaggcc cctacgggct actacaaccc ttcgctgacg ccataaaact cttcaccaaa 3480

gagcccctaa aacccgccac atctaccatc accctctaca tcaccgcccc gaccttagct 3540

ctcaccatcg ctcttctact atgaaccccc ctccccatac ccaaccccct ggtcaacctc 3600

aacctaggcc tcctatttat tctagccacc tctagcctag ccgtttactc aatcctctga 3660

tcagggtgag catcaaactc aaactacgcc ctgatcggcg cactgcgagc agtagcccaa 3720

acaatctcat atgaagtcac cctagccatc attctactat caacattact aataagtggc 3780

tcctttaacc tctccaccct tatcacaaca caagaacacc tctgattact cctgccatca 3840

tgacccttgg ccataatatg atttatctcc acactagcag agaccaaccg aacccccttc 3900

gaccttgccg aaggggagtc cgaactagtc tcaggcttca acatcgaata cgccgcaggc 3960

cccttcgccc tattcttcat agccgaatac acaaacatta ttataataaa caccctcacc 4020

actacaatct tcctaggaac aacatatgac gcactctccc ctgaactcta cacaacatat 4080

tttgtcacca agaccctact tctaacctcc ctgttcttat gaattcgaac agcatacccc 4140

cgattccgct acgaccaact catacacctc ctatgaaaaa acttcctacc actcacccta 4200

gcattactta tatgatatgt ctccataccc attacaatct ccagcattcc ccctcaaacc 4260

taagaaatat gtctgataaa agagttactt tgatagagta aataatagga gcttaaaccc 4320

ccttatttct aggactatga gaatcgaacc catccctgag aatccaaaat tctccgtgcc 4380

acctatcaca ccccatccta aagtaaggtc agctaaataa gctatcgggc ccataccccg 4440

aaaatgttgg ttataccctt cccgtactaa ttaatcccct ggcccaaccc gtcatctact 4500

ctaccatctt tgcaggcaca ctcatcacag cgctaagctc gcactgattt tttacctgag 4560

taggcctaga aataaacatg ctagctttta ttccagttct aaccaaaaaa ataaaccctc 4620

gttccacaga agctgccatc aagtatttcc tcacgcaagc aaccgcatcc ataatccttc 4680

taatagctat cctcttcaac aatatactct ccggacaatg aaccataacc aatactacca 4740

atcaatactc atcattaata atcataatag ctatagcaat aaaactagga atagccccct 4800

ttcacttctg agtcccagag gttacccaag gcacccctct gacatccggc ctgcttcttc 4860

tcacatgaca aaaactagcc cccatctcaa tcatatacca aatctctccc tcactaaacg 4920

taagccttct cctcactctc tcaatcttat ccatcatagc aggcagttga ggtggattaa 4980

accaaaccca gctacgcaaa atcttagcat actcctcaat tacccacata ggatgaataa 5040

tagcagttct accgtacaac cctaacataa ccattcttaa tttaactatt tatattatcc 5100

taactactac cgcattccta ctactcaact taaactccag caccacgacc ctactactat 5160

ctcgcacctg aaacaagcta acatgactaa cacccttaat tccatccacc ctcctctccc 5220

taggaggcct gcccccgcta accggctttt tgcccaaatg ggccattatc gaagaattca 5280

caaaaaacaa tagcctcatc atccccacca tcatagccac catcaccctc cttaacctct 5340

acttctacct acgcctaatc tactccacct caatcacact actccccata tctaacaacg 5400

taaaaataaa atgacagttt gaacatacaa aacccacccc attcctcccc acactcatcg 5460

cccttaccac gctactccta cctatctccc cttttatact aataatctta tagaaattta 5520

ggttaaatac agaccaagag ccttcaaagc cctcagtaag ttgcaatact taatttctgt 5580

aacagctaag gactgcaaaa ccccactctg catcaactga acgcaaatca gccactttaa 5640

ttaagctaag cccttactag accaatggga cttaaaccca caaacactta gttaacagct 5700

aagcacccta atcaactggc ttcaatctac ttctcccgcc gccgggaaaa aaggcgggag 5760

aagccccggc aggtttgaag ctgcttcttc gaatttgcaa ttcaatatga aaatcacctc 5820

ggagctggta aaaagaggcc taacccctgt ctttagattt acagtccaat gcttcactca 5880

gccattttac ctcaccccca ctgatgttcg ccgaccgttg actattctct acaaaccaca 5940

aagacattgg aacactatac ctattattcg gcgcatgagc tggagtccta ggcacagctc 6000

taagcctcct tattcgagcc gagctgggcc agccaggcaa ccttctaggt aacgaccaca 6060

tctacaacgt tatcgtcaca gcccatgcat ttgtaataat cttcttcata gtaataccca 6120

tcataatcgg aggctttggc aactgactag ttcccctaat aatcggtgcc cccgatatgg 6180

cgtttccccg cataaacaac ataagcttct gactcttacc tccctctctc ctactcctgc 6240

tcgcatctgc tatagtggag gccggagcag gaacaggttg aacagtctac cctcccttag 6300

cagggaacta ctcccaccct ggagcctccg tagacctaac catcttctcc ttacacctag 6360

caggtgtctc ctctatctta ggggccatca atttcatcac aacaattatc aatataaaac 6420

cccctgccat aacccaatac caaacgcccc tcttcgtctg atccgtccta atcacagcag 6480

tcctacttct cctatctctc ccagtcctag ctgctggcat cactatacta ctaacagacc 6540

gcaacctcaa caccaccttc ttcgaccccg ccggaggagg agaccccatt ctataccaac 6600

acctattctg atttttcggt caccctgaag tttatattct tatcctacca ggcttcggaa 6660

taatctccca tattgtaact tactactccg gaaaaaaaga accatttgga tacataggta 6720

tggtctgagc tatgatatca attggcttcc tagggtttat cgtgtgagca caccatatat 6780

ttacagtagg aatagacgta gacacacgag catatttcac ctccgctacc ataatcatcg 6840

ctatccccac cggcgtcaaa gtatttagct gactcgccac actccacgga agcaatatga 6900

aatgatctgc tgcagtgctc tgagccctag gattcatctt tcttttcacc gtaggtggcc 6960

tgactggcat tgtattagca aactcatcac tagacatcgt actacacgac acgtactacg 7020

ttgtagccca cttccactat gtcctatcaa taggagctgt atttgccatc ataggaggct 7080

tcattcactg atttccccta ttctcaggct acaccctaga ccaaacctac gccaaaatcc 7140

atttcactat catattcatc ggcgtaaatc taactttctt cccacaacac tttctcggcc 7200

tatccggaat gccccgacgt tactcggact accccgatgc atacaccaca tgaaacatcc 7260

tatcatctgt aggctcattc atttctctaa cagcagtaat attaataatt ttcatgattt 7320

gagaagcctt cgcttcgaag cgaaaagtcc taatagtaga agaaccctcc ataaacctgg 7380

agtgactata tggatgcccc ccaccctacc acacattcga agaacccgta tacataaaat 7440

ctagacaaaa aaggaaggaa tcgaaccccc caaagctggt ttcaagccaa ccccatggcc 7500

tccatgactt tttcaaaaag gtattagaaa aaccatttca taactttgtc aaagttaaat 7560

tataggctaa atcctatata tcttaatggc acatgcagcg caagtaggtc tacaagacgc 7620

tacttcccct atcatagaag agcttatcac ctttcatgat cacgccctca taatcatttt 7680

ccttatctgc ttcctagtcc tgtatgccct tttcctaaca ctcacaacaa aactaactaa 7740

tactaacatc tcagacgctc aggaaataga aaccgtctga actatcctgc ccgccatcat 7800

cctagtcctc atcgccctcc catccctacg catcctttac ataacagacg aggtcaacga 7860

tccctccctt accatcaaat caattggcca ccaatggtac tgaacctacg agtacaccga 7920

ctacggcgga ctaatcttca actcctacat acttccccca ttattcctag aaccaggcga 7980

cctgcgactc cttgacgttg acaatcgagt agtactcccg attgaagccc ccattcgtat 8040

aataattaca tcacaagacg tcttgcactc atgagctgtc cccacattag gcttaaaaac 8100

agatgcaatt cccggacgtc taaaccaaac cactttcacc gctacacgac cgggggtata 8160

ctacggtcaa tgctctgaaa tctgtggagc aaaccacagt ttcatgccca tcgtcctaga 8220

attaattccc ctaaaaatct ttgaaatagg gcccgtattt accctatagc accccctcta 8280

ccccctctag agcccactgt aaagctaact tagcattaac cttttaagtt aaagattaag 8340

agaaccaaca cctctttaca gtgaaatgcc ccaactaaat actaccgtat ggcccaccat 8400

aattaccccc atactcctta cactattcct catcacccaa ctaaaaatat taaacacaaa 8460

ctaccaccta cctccctcac caaagcccat aaaaataaaa aattataaca aaccctgaga 8520

accaaaatga acgaaaatct gttcgcttca ttcattgccc ccacaatcct aggcctaccc 8580

gccgcagtac tgatcattct atttccccct ctattgatcc ccacctccaa atatctcatc 8640

aacaaccgac taatcaccac ccaacaatga ctaatcaaac taacctcaaa acaaatgata 8700

accatacaca acactaaagg acgaacctga tctcttatac tagtatcctt aatcattttt 8760

attgccacaa ctaacctcct cggactcctg cctcactcat ttacaccaac cacccaacta 8820

tctataaacc tagccatggc catcccctta tgagcgggca cagtgattat aggctttcgc 8880

tctaagatta aaaatgccct agcccacttc ttaccacaag gcacacctac accccttatc 8940

cccatactag ttattatcga aaccatcagc ctactcattc aaccaatagc cctggccgta 9000

cgcctaaccg ctaacattac tgcaggccac ctactcatgc acctaattgg aagcgccacc 9060

ctagcaatat caaccattaa ccttccctct acacttatca tcttcacaat tctaattcta 9120

ctgactatcc tagaaatcgc tgtcgcctta atccaagcct acgttttcac acttctagta 9180

agcctctacc tgcacgacaa cacataatga cccaccaatc acatgcctat catatagtaa 9240

aacccagccc atgaccccta acaggggccc tctcagccct cctaatgacc tccggcctag 9300

ccatgtgatt tcacttccac tccataacgc tcctcatact aggcctacta accaacacac 9360

taaccatata ccaatgatgg cgcgatgtaa cacgagaaag cacataccaa ggccaccaca 9420

caccacctgt ccaaaaaggc cttcgatacg ggataatcct atttattacc tcagaagttt 9480

ttttcttcgc aggatttttc tgagcctttt accactccag cctagcccct accccccaat 9540

taggagggca ctggccccca acaggcatca ccccgctaaa tcccctagaa gtcccactcc 9600

taaacacatc cgtattactc gcatcaggag tatcaatcac ctgagctcac catagtctaa 9660

tagaaaacaa ccgaaaccaa ataattcaag cactgcttat tacaatttta ctgggtctct 9720

attttaccct cctacaagcc tcagagtact tcgagtctcc cttcaccatt tccgacggca 9780

tctacggctc aacatttttt gtagccacag gcttccacgg acttcacgtc attattggct 9840

caactttcct cactatctgc ttcatccgcc aactaatatt tcactttaca tccaaacatc 9900

actttggctt cgaagccgcc gcctgatact ggcattttgt agatgtggtt tgactatttc 9960

tgtatgtctc catctattga tgagggtctt actcttttag tataaatagt accgttaact 10020

tccaattaac tagttttgac aacattcaaa aaagagtaat aaacttcgcc ttaattttaa 10080

taatcaacac cctcctagcc ttactactaa taattattac attttgacta ccacaactca 10140

acggctacat agaaaaatcc accccttacg agtgcggctt cgaccctata tcccccgccc 10200

gcgtcccttt ctccataaaa ttcttcttag tagctattac cttcttatta tttgatctag 10260

aaattgccct ccttttaccc ctaccatgag ccctacaaac aactaacctg ccactaatag 10320

ttatgtcatc cctcttatta atcatcatcc tagccctaag tctggcctat gagtgactac 10380

aaaaaggatt agactgaacc gaattggtat atagtttaaa caaaacgaat gatttcgact 10440

cattaaatta tgataatcat atttaccaaa tgcccctcat ttacataaat attatactag 10500

catttaccat ctcacttcta ggaatactag tatatcgctc acacctcata tcctccctac 10560

tatgcctaga aggaataata ctatcgctgt tcattatagc tactctcata accctcaaca 10620

cccactccct cttagccaat attgtgccta ttgccatact agtctttgcc gcctgcgaag 10680

cagcggtggg cctagcccta ctagtctcaa tctccaacac atatggccta gactacgtac 10740

ataacctaaa cctactccaa tgctaaaact aatcgtccca acaattatat tactaccact 10800

gacatgactt tccaaaaaac acataatttg aatcaacaca accacccaca gcctaattat 10860

tagcatcatc cctctactat tttttaacca aatcaacaac aacctattta gctgttcccc 10920

aaccttttcc tccgaccccc taacaacccc cctcctaata ctaactacct gactcctacc 10980

cctcacaatc atggcaagcc aacgccactt atccagtgaa ccactatcac gaaaaaaact 11040

ctacctctct atactaatct ccctacaaat ctccttaatt ataacattca cagccacaga 11100

actaatcata ttttatatct tcttcgaaac cacacttatc cccaccttgg ctatcatcac 11160

ccgatgaggc aaccagccag aacgcctgaa cgcaggcaca tacttcctat tctacaccct 11220

agtaggctcc cttcccctac tcatcgcact aatttacact cacaacaccc taggctcact 11280

aaacattcta ctactcactc tcactgccca agaactatca aactcctgag ccaacaactt 11340

aatatgacta gcttacacaa tagcttttat agtaaagata cctctttacg gactccactt 11400

atgactccct aaagcccatg tcgaagcccc catcgctggg tcaatagtac ttgccgcagt 11460

actcttaaaa ctaggcggct atggtataat acgcctcaca ctcattctca accccctgac 11520

aaaacacata gcctacccct tccttgtact atccctatga ggcataatta taacaagctc 11580

catctgccta cgacaaacag acctaaaatc gctcattgca tactcttcaa tcagccacat 11640

agccctcgta gtaacagcca ttctcatcca aaccccctga agcttcaccg gcgcagtcat 11700

tctcataatc gcccacgggc ttacatcctc attactattc tgcctagcaa actcaaacta 11760

cgaacgcact cacagtcgca tcataatcct ctctcaagga cttcaaactc tactcccact 11820

aatagctttt tgatgacttc tagcaagcct cgctaacctc gccttacccc ccactattaa 11880

cctactggga gaactctctg tgctagtaac cacgttctcc tgatcaaata tcactctcct 11940

acttacagga ctcaacatac tagtcacagc cctatactcc ctctacatat ttaccacaac 12000

acaatggggc tcactcaccc accacattaa caacataaaa ccctcattca cacgagaaaa 12060

caccctcatg ttcatacacc tatcccccat tctcctccta tccctcaacc ccgacatcat 12120

taccgggttt tcctcttgta aatatagttt aaccaaaaca tcagattgtg aatctgacaa 12180

cagaggctta cgacccctta tttaccgaga aagctcacaa gaactgctaa ctcatgcccc 12240

catgtctaac aacatggctt tctcaacttt taaaggataa cagctatcca ttggtcttag 12300

gccccaaaaa ttttggtgca actccaaata aaagtaataa ccatgcacac tactataacc 12360

accctaaccc tgacttccct aattcccccc atccttacca ccctcgttaa ccctaacaaa 12420

aaaaactcat acccccatta tgtaaaatcc attgtcgcat ccacctttat tatcagtctc 12480

ttccccacaa caatattcat gtgcctagac caagaagtta ttatctcgaa ctgacactga 12540

gccacaaccc aaacaaccca gctctcccta agcttcaaac tagactactt ctccataata 12600

ttcatccctg tagcattgtt cgttacatgg tccatcatag aattctcact gtgatatata 12660

aactcagacc caaacattaa tcagttcttc aaatatctac tcatcttcct aattaccata 12720

ctaatcttag ttaccgctaa caacctattc caactgttca tcggctgaga gggcgtagga 12780

attatatcct tcttgctcat cagttgatga tacgcccgag cagatgccaa cacagcagcc 12840

attcaagcaa tcctatacaa ccgtatcggc gatatcggtt tcatcctcgc cttagcatga 12900

tttatcctac actccaactc atgagaccca caacaaatag cccttctaaa cgctaatcca 12960

agcctcaccc cactactagg cctcctccta gcagcagcag gcaaatcagc ccaattaggt 13020

ctccacccct gactcccctc agccatagaa ggccccaccc cagtctcagc cctactccac 13080

tcaagcacta tagttgtagc aggaatcttc ttactcatcc gcttccaccc cctagcagaa 13140

aatagcccac taatccaaac tctaacacta tgcttaggcg ctatcaccac tctgttcgca 13200

gcagtctgcg cccttacaca aaatgacatc aaaaaaatcg tagccttctc cacttcaagt 13260

caactaggac tcataatagt tacaatcggc atcaaccaac cacacctagc attcctgcac 13320

atctgtaccc acgccttctt caaagccata ctatttatgt gctccgggtc catcatccac 13380

aaccttaaca atgaacaaga tattcgaaaa ataggaggac tactcaaaac catacctctc 13440

acttcaacct ccctcaccat tggcagccta gcattagcag gaataccttt cctcacaggt 13500

ttctactcca aagaccacat catcgaaacc gcaaacatat catacacaaa cgcctgagcc 13560

ctatctatta ctctcatcgc tacctccctg acaagcgcct atagcactcg aataattctt 13620

ctcaccctaa caggtcaacc tcgcttcccc acccttacta acattaacga aaataacccc 13680

accctactaa accccattaa acgcctggca gccggaagcc tattcgcagg atttctcatt 13740

actaacaaca tttcccccgc atcccccttc caaacaacaa tccccctcta cctaaaactc 13800

acagccctcg ctgtcacttt cctaggactt ctaacagccc tagacctcaa ctacctaacc 13860

aacaaactta aaataaaatc cccactatgc acattttatt tctccaacat actcggattc 13920

taccctagca tcacacaccg cacaatcccc tatctaggcc ttcttacgag ccaaaacctg 13980

cccctactcc tcctagacct aacctgacta gaaaagctat tacctaaaac aatttcacag 14040

caccaaatct ccacctccat catcacctca acccaaaaag gcataattaa actttacttc 14100

ctctctttct tcttcccact catcctaacc ctactcctaa tcacataacc tattcccccg 14160

agcaatctca attacaatat atacaccaac aaacaatgtt caaccagtaa ctactactaa 14220

tcaacgccca taatcataca aagcccccgc accaatagga tcctcccgaa tcaaccctga 14280

cccctctcct tcataaatta ttcagcttcc tacactatta aagtttacca caaccaccac 14340

cccatcatac tctttcaccc acagcaccaa tcctacctcc atcgctaacc ccactaaaac 14400

actcaccaag acctcaaccc ctgaccccca tgcctcagga tactcctcaa tagccatcgc 14460

tgtagtatat ccaaagacaa ccatcattcc ccctaaataa attaaaaaaa ctattaaacc 14520

catataacct cccccaaaat tcagaataat aacacacccg accacaccgc taacaatcaa 14580

tactaaaccc ccataaatag gagaaggctt agaagaaaac cccacaaacc ccattactaa 14640

acccacactc aacagaaaca aagcatacat cattattctc gcacggacta caaccacgac 14700

caatgatatg aaaaaccatc gttgtatttc aactacaaga acaccaatga ccccaatacg 14760

caaaactaac cccctaataa aattaattaa ccactcattc atcgacctcc ccaccccatc 14820

caacatctcc gcatgatgaa acttcggctc actccttggc gcctgcctga tcctccaaat 14880

caccacagga ctattcctag ccatgcacta ctcaccagac gcctcaaccg ccttttcatc 14940

aatcgcccac atcactcgag acgtaaatta tggctgaatc atccgctacc ttcacgccaa 15000

tggcgcctca atattcttta tctgcctctt cctacacatc gggcgaggcc tatattacgg 15060

atcatttctc tactcagaaa cctgaaacat cggcattatc ctcctgcttg caactatagc 15120

aacagccttc ataggctatg tcctcccgtg aggccaaata tcattctgag gggccacagt 15180

aattacaaac ttactatccg ccatcccata cattgggaca gacctagttc aatgaatctg 15240

aggaggctac tcagtagaca gtcccaccct cacacgattc tttacctttc acttcatctt 15300

gcccttcatt attgcagccc tagcaacact ccacctccta ttcttgcacg aaacgggatc 15360

aaacaacccc ctaggaatca cctcccattc cgataaaatc accttccacc cttactacac 15420

aatcaaagac gccctcggct tacttctctt ccttctctcc ttaatgacat taacactatt 15480

ctcaccagac ctcctaggcg acccagacaa ttatacccta gccaacccct taaacacccc 15540

tccccacatc aagcccgaat gatatttcct attcgcctac acaattctcc gatccgtccc 15600

taacaaacta ggaggcgtcc ttgccctatt actatccatc ctcatcctag caataatccc 15660

catcctccat atatccaaac aacaaagcat aatatttcgc ccactaagcc aatcacttta 15720

ttgactccta gccgcagacc tcctcattct aacctgaatc ggaggacaac cagtaagcta 15780

cccttttacc atcattggac aagtagcatc cgtactatac ttcacaacaa tcctaatcct 15840

aataccaact atctccctaa ttgaaaacaa aatactcaaa tgggcctgtc cttgtagtat 15900

aaactaatac accagtcttg taaaccggag atgaaaacct ttttccaagg acaaatcaga 15960

gaaaaagtct ttaactccac cattagcacc caaagctaag attctaattt aaactattct 16020

ctgttctttc atggggaagc agatttgggt accacccaag tattgactca cccatcaaca 16080

accgctatgt atttcgtaca ttactgccag ccaccatgaa tattgtacgg taccataaat 16140

acttgaccac ctgtagtaca taaaaaccca atccacatca aaaccccctc cccatgctta 16200

caagcaagta cagcaatcaa ccctcaacta tcacacatca actgcaactc caaagccacc 16260

cctcacccac taggatacca acaaacctac ccacccttaa cagtacatag tacataaagc 16320

catttaccgt acatagcaca ttacagtcaa atcccttctc gtccccatgg atgacccccc 16380

tcagataggg gtcccttgac caccatcctc cgtgaaatca atatcccgca caagagtgct 16440

actctcctcg ctccgggccc ataacacttg ggggtagcta aagtgaactg tatccgacat 16500

ctggttccta cttcagggtc ataaagccta aatagcccac acgttcccct taaataagac 16560

atcacgatg 16569

<210> 7
<211> 21
<212> DNA
<213> Artificial

<220>
<223> forward primer

<400> 7
cacccatcaa caaccgctat g 21

<210> 8
<211> 22
<212> DNA
<213> Artificial

<220>
<223> reverse primer

<400> 8
ggatgaggca ggaatcaaag ac 22

<210> 9
<211> 652
<212> DNA
<213> Homo sapiens

<400> 9
cacccatcaa caaccgctat gtatttcgta cattactgcc agccaccatg aatattgtac 60

ggtaccataa atacttgacc acctgtagta cataaaaacc caatccacat caaaaccccc 120

tccccatgct tacaagcaag tacagcaatc aaccctcaac tatcacacat caactgcaac 180

tccaaagcca cccctcaccc actaggatac caacaaacct acccaccctt aacagtacat 240

agtacataaa gccatttacc gtacatagca cattacagtc aaatcccttc tcgtccccat 300

ggatgacccc cctcagatag gggtcccttg accaccatcc tccgtgaaat caatatcccg 360

cacaagagtg ctactctcct cgctccgggc ccataacact tgggggtagc taaagtgaac 420

tgtatccgac atctggttcc tacttcaggg tcataaagcc taaatagccc acacgttccc 480

cttaaataag acatcacgat ggatcacagg tctatcaccc tattaaccac tcacgggagc 540

tctccatgca tttggtattt tcgtctgggg ggtatgcacg cgatagcatt gcgagacgct 600

ggagccggag caccctatgt cgcagtatct gtctttgatt cctgcctcat cc 652

## Description

### Technical Field

The present invention is framed within the health field and relates to methods and markers for evaluating the risk of suffering a cardiovascular disease.

### Background Art

Cardiovascular disease (CVD) is one of the most widespread and common causes of death in the world. Particularly, stroke and myocardial infarction (Ml) are complex disorders that share a common pathophysiology involving atherosclerosis and thrombosis (or clotting). The onset and severity of these diseases are influenced by both genetic and environmental factors. Recent evidences associate mitochondrial dysfunction with several cardiovascular manifestations, mainly driven by the central role of mitochondria in cellular metabolism, particularly in energetically demanding tissues such as the brain and heart (see Taverne et al.2013 "Reactive oxygen species and the cardiovascular system. Oxid Med Cell Longev 2013, 862423)

Classical risk factors for CVD (such as Ml and stroke) include clinical parameters or clinical variables. Examples of these are hypertension, diabetes or hypercholesterolemia. -. Other variables relate to age, sex - and certain habits (i.e. smoking).

Also genetic factors have been associated with particular cardiovascular disease onsets, in particular with coronary heart disease, where several single nucleotide polymorphisms (SNPs) in nuclear DNA (nDNA) have been correlated with a high risk of suffering from the disease (see Beaney et al., "How close are we to implementing a genetic risk score for coronary heart disease?", Expert Review of Molecular Diagnostics 2017, DOI: 10.1080/14737159.2017.1368388; and Christiansen et al. "A genetic risk score predicts cardiovascular events in patients with stable coronary artery disease", International Journal of Cardiology 2017, http://dx.doi.org/10.1016/j.ijcard.2017.04.045). In addition, most reports have attempted to evaluate whether the use of a nuclear genetic risk score (GRS) through several established CVD risk algorithm, such as the Framingham risk score or its different region-specific scores as REGICOR for Spain population, would improve disease prediction beyond of classical variable risk factors (CVFRs). For this purpose, cardiovascular risk DNA-chip, Cardio inCode® (Gendiag, Barcelona, Spain), was the first test designed to predict cardiovascular risk using a genetic score that incorporates information from 11 nuclear variants associated with CVD (see Lluís-Ganella et al. "Additive effect of multiple genetic variants on the risk of coronary artery disease", Rev Esp Cardiol-2010, vol no. 63, pp:925-933; and Lluís-Ganella et al., "Assessment of the value of a genetic risk score in improving the estimation of coronary risk", Atherosclerosis -. 2012, vol no 22, . pp:456-463) but not related to classic risk factors.

Recent evidence have linked certain CVDs with specific mitochondrial DNA (mtDNA) variants including base substitution, deletions, duplications and point or length heteroplasmy both in coding and noncoding region of mtDNA. In particular, mtDNA variants located in control region (CR) also known as D-loop have a potential importance since they may influence on the regulation of the mtDNA gene expression. In fact, several studies detected association of a great range of mtDNA variants (with negative or beneficial effect, both fixed or in heteroplasmy) and different diseases (see Hudson et al., "Recent Mitochondrial DNA Mutations Increase the Risk of Developing Common Late-Onset Human Diseases", PLoS Genet 10 2014, e1004369; Wang et al. "Cardiovascular Disease, Mitochondria, and Traditional Chinese Medicine". Evidence-Based Complementary and Alternative Medicine 2015, 7; and Gawe 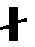 et al., "The G16319A substitution frequency in a hemorrhagic stroke", Annals of Indian Academy of Neurology 2008, vol. no. 11, pp.:154-158.)

Human mtDNA is 16.6-kb double-stranded circular DNA molecule that encodes for 13 electron transport chain (ETC) proteins, 2 ribosomal RNAs (rRNAs) and 22 transports RNAs (tRNAs). Sequence of human mtDNA is the GenBank accession number NC_012920, version 1 of 31-OCT-2014 (https://www.ncbi.nlm.nih.gov/nuccore/NC 012920.1). This polynucleotide sequence is known as the revised Cambridge rReference Sequence (rCRS). rCRS is also disclosed in Andrews, et al., "Reanalysis and revision of the Cambridge reference sequence for human mitochondrial DNA" Nature genetics,1999, vol. no. 23, p.147. In this description is also listed as SEQ ID NO: 6.The CR of mtDNA encompasses the light and heavy strand promoters, the heavy strand origin of replication (OH), three conserved sequence blocks and the termination associated sequences (TAS). CR also known as D-loop corresponds to a polynucleotide sequence, defined in sequence of NC_012920 accession number as join of bases 16024..16569,1..576 of the sequence. MtDNA is more susceptible than nuclear DNA to oxidative damage, probably due to the lack of histone complex and an inefficient DNA repair mechanisms, which may serve as a protective barrier against external and internal noxious agents as reactive oxygen species (ROS). Furthermore, the close proximity of mtDNA to the ETC, the major source of ROS production, could also contribute to enhanced susceptibility of mtDNA to oxidative damage and could explain its high mutation rate (∼10-fold greater than in nDNA). Hence, mtDNA mutations are very common and increase with age.

Although many attempts have been performed in order to evaluate the risk of a patient of suffering from CVD, there is still a need of alternative methods aiming to have methods for particular populations or cohorts of patients, as well as a need of additional markers to assure a right classification of as much subjects as possible. Methods with improved discrimination and calibration are needed since with the techniques nowadays in use around 60% of myocardial infarctions are diagnosed in subjects classified as low risk for myocardial infarction according to clinical practise.

### Summary of Invention

Inventors surprisingly found that SNPs in mtDNA, all of them in the region of mtDNA known as D-loop, are related with the risk of suffering from CVD. A risk score with the newly identified SNPs was determined and it allowed a high discrimination capability (measured in ROC curves), which predictability even increased when in the calculation/computing of the risk score clinical variables and additional SNPs from nDNA where taken in consideration.

Thus, a first aspect of the invention is an *in vitro* method for evaluating the risk of suffering from a CVD, the method comprising determining in an isolated sample of a subject, if one or more genetic variants are present in D-loop sequence of mtDNA, said mtDNA being that of SEQ ID NO: 6 (or rCRS), wherein the genetic variants are selected from one or more of 16145G>A, 16311T>C, 16356T>C. 72T>C and 73A>G.

These variants are referred in this description as SNPs or also as genetic variants. The notation indicates the nucleotide number or position of the sequence of human mtDNA, in which a change has occurred, being the position given according to rCRS disclosed above. Nucleotide in rCRS is indicated first, and the nucleotide for which is substituted is indicated further after >. Therefore, 16145G>A means that at position 16145 of rCRS of mtDNA a guanine (G) has been replaced by an adenine (A). Other equivalent SNPs notation used is G16145A or m.16145G>A.

A further aspect of the invention is a set of primers comprising, and more particularly consisting of, forward primer 5'-CACCCATCAACAACCGCTATG-3' (SEQ ID NO: 7) and reverse primer 5'-GGATGAGGCAGGAATCAAAGAC-3' (SEQ ID NO: 8)

This set of primers allows amplifying a fragment of the D-loop of mtDNA comprising the nucleotide changes resulting in the genetic variants determined according to the present invention. This fragment of D-loop is defined by the continuous sequence encompassing from nucleotide 16069 to 16569 and from nucleotide 1 to 151 of rCRS and is also defined in this description as SEQ ID NO: 9. They are thus related with the novel SNPs variations related with CVD risk identified by the inventors.

These primers allow fast amplification of the zone of interest, since with a single specific amplification a polynucleotide fragment of standard length for Sanger sequencing is obtained. Hence, fast and simple identification of the genetic variants is accomplished.

Another aspect of the invention is a method of deciding or recommending whether to initiate a prophylactic and/or therapeutic regimen of a subject suspected at risk of suffering from a CVD, which method comprises:
a) detecting, in vitro, according to the method disclosed in the first aspect, if one or more genetic variants are present in D-loop of mtDNA from an isolated sample of a subject, wherein the variants are selected from one or more of 16145G>A, 16311T>C, 16356T>C, 72T>C and 73A>G and determining a risk score of suffering from a CVD; and
b) if the subject is considered at risk of suffering from a CVD, recommending a prophylactic and/or therapeutic regimen.

### Detailed description of the invention

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

The sense of the invention, the expression a "method comprising determining if one or more variants are present" encompasses detecting, confirming presence or absence of a particular variant in a nucleotide sequence (mtDNA sequence or nDNA sequence) that is correlated with the risk of a subject of suffering a CVD.

For "reference individual or control individual (simply control)" it is to be understood as a subject or group of subjects wherein the presence, absence, stage, or course of the disease has been properly performed previously. The subject or subjects from a "control population" may include subject/s wherein the condition is absent, subject/s wherein the condition is present, or both. The skilled person in the art, making use of the general knowledge, is able to choose the subject or group of subjects more adequate for obtaining the reference value for each of the methods of the present invention. In a particular embodiment, and as disclosed in the examples below, the controls are subjects not suffering from a cardiovascular disease, in particular myocardial infarct or stroke.

The term "risk score" (or risk scoring) is the name given to a general practice in applied statistics, bio-statistics, econometrics and other related disciplines, of creating an easily calculated number (the score) that reflects the level of risk in the presence of some risk factors (e.g. risk of suffering from a CVD (MI or stroke) in the presence of symptoms or genetic profile, age, presence of other diseases or clinical variables, such as hypercholesterolemia and hypertension, etc.). In the particular case of cardiovascular risk, "cardiovascular risk scoring systems" give an estimate of the probability that a person will develop cardiovascular disease within a specified amount of time, usually 10 to 30 years. Because they give an indication of the risk of developing cardiovascular disease, they also indicate who is most likely to benefit from prevention. For this reason, cardiovascular risk scores are used to determine who should be offered preventive drugs such as drugs to lower blood pressure and drugs to lower cholesterol levels. Many are the algorithms and statistical models for generating risk scores. One example includes conditional logistic regression. "Conditional logistic regression" is an extension of logistic regression that allows one to take into account stratification and matching. Its main field of application is observational studies and in particular epidemiology. It was designed in 1978 and is widely known by skilled man. It is the most flexible and general procedure for matched data. Examples of known algorithms for cardiovascular risk scoring include the Framingham Risk Score that determines an individual's chances of developing cardiovascular disease in 10 years; and the REGICOR, which is a function derived from that of Framingham Risk Score and that has been validated and calibrated for Spanish population. Other algorithms for risk of CVD are available on-line and are well known by the skilled man.

"Genetic risk score (GRS)" or "genetic score" is to be understood as a risk score in which genetic parameters (mainly genetic variants) are taken in consideration when evaluating a particular level of risk.

The term "single-nucleotide polymorphism", often abbreviated to SNP, and also termed in this description as "variant" or "genetic variant", is a variation in a single nucleotide that occurs at a specific position in the genome, where each variation is present to some appreciable degree within a population (e.g. > 1%). In some occasions, prior art also refers with SNP few nucleotide changes, as well as small deletions or insertions (indels) if they are with a frequency of > 1% in a population.

"Discrimination" is the treatment or consideration of a person based on the group or category to which the person is perceived to belong, in this description the category of being at risk of suffering a CVD or not. Thus, the discrimination capability is the capability of correctly classifying a subject between a group with risk of CVD and a group without a risk of CVD.

In the present invention, the expression "prophylactic and/or therapeutic regimen" is a synonymous of "medical regimen", and is to be understood as encompassing either pharmacological therapies (such as the administration of statins) as well as other clinical decisions taken by a physician (such as surgery), among others.

As above indicated, the invention relates to an in vitro method for evaluating the risk of suffering from a cardiovascular disease, the method comprising determining the presence or absence of one or more genetic variants in D-loop of mtDNA (of sequence rCRS) from an isolated sample of a subject, said genetic variants selected from one or more of 16145G>A, 16311T>C, 16356T>C, 72T>C and 73A>G.

In a particular embodiment of the method of the first aspect, if none, one or more mutations are detected then a risk score is calculated and it is concluded if the calculated risk score is indicative of risk of suffering from CVD or not, optionally grading the risk if any.

In a particular embodiment of the first aspect, the CVD is selected from stroke, myocardial infarct (including fatal or non-fatal myocardial infract), angina pectoris, coronary revascularization, acute coronary syndrome, peripheral arterial disease (also named peripheral arteriopathy) and combinations thereof. In a more particular embodiment, the CVD is stroke. In another more particular embodiment the CVD is myocardial infarct said myocardial infract including any of fatal or non-fatal myocardial infarct.

In another particular embodiment of the first aspect, the method comprises determining if there are none, at least one, at least two, at least three, at least four or the five of the genetic variants 16145G>A, 16311T>C, 16356T>C, 72T>C and73A>G. In a more particular embodiment, the method comprises determining if there are the five variants in D-loop of human mtDNA of SEQ ID NO: 6, or also referred as rCRS.

Inventors also determined that some of the above listed SNPs emerged as a possible genetic risk factors for stroke, as a particular onset of CVD. Therefore, in another particular embodiment of the first aspect, optionally in combination with any embodiments above or below, if at least one of 16145G>A, and 16311T>C is detected, it is concluded that the subject comprises a variant increasing probability of suffering from at least stroke. In yet a more particular embodiment, if none, one or more variants from 16145G>A, and 16311T>C are detected, then a risk score is further calculated or computed using particular formulas and/or algorithms for CVD risk scoring.

On the other hand, and according to another particular embodiment of the in vitro method of the first aspect, if at least one of 72T>C, 73A>G and 16356T>C is detected, it is concluded that the subject comprises beneficial variants protecting from suffering from a myocardial infarct.

In the same way as for stroke risk variants, if none, one or more variants from 72T>C, 73A>G and 16356T>C are detected, then a risk score is further calculated or computed using particular formulas and/or algorithms for CVD risk scoring.

Risk scores are, in a particular embodiment, combined with other risk scores also aiming the correlation of an event with the risk of suffering from a CVD.

Several parameters currently used in the evaluation of the risk of suffering from a CVD were tested by inventors in combination with the above listed variants in mtDNA. It was surprisingly found that area under curve (AUC) in ROC analysis was improved in a meaningful way when clinical variables, and/or a GRS determined from nDNA variants) were combined or pondered with the mtDNA variants in D-loop above.

Thus, in another particular embodiment of the first aspect, the method further comprises determining in the isolated sample of the subject one or more nDNA variants. Thus, the method further comprises determining in the isolated sample of the subject the genotype of one or more nDNA genetic variants.

In a more particular embodiment, nDNA variants are selected from the group consisting of rs10507391, rs17222842, rs9315051, rs1333049, rs1746048, rs9982601, rs10455872, rs17465637, rs9818870, rs12526453, rs6725887. These nDNA variants or SNPs correspond with the notation of SNPs database dbSNP of the National Center for Biotechnology Information (NCBI). They can be retrieved from https://www.ncbi.nlm.nih.gov/snp/

In a more particular embodiment of the first aspect, optionally in combination with any embodiment above or below, presence or absence of two, three, four, five, six, seven, eight, nine, ten or the eleven of nDNA variants rs10507391, rs17222842, rs9315051, rs1333049, rs1746048, rs9982601, rs10455872, rs17465637, rs9818870, rs12526453, rs6725887 is determined in the isolated sample of the subject.

The above nDNA genetic variants are the ones in test known as Cardio inCode® (Gendiag, Barcelona, Spain).

Independently if they are genetic variants in mtDNA or in nDNA, in a particular embodiment of the first aspect said SNPs are determined by means of genotype methods. Particular genotype methods are selected from Sanger sequencing, pyrosequencing, hybridation, restriction fragment lenght polynorphism (RFLP), polymerase chain reaction (PCR), Amplified fragment length polymorphism (AFLP), and combinations thereof. Examples of pyrosequencing genotype methods include, among others, the next generation sequencing (NGS) methods known as 454 High though output pyrosequencing, sequencing by synthesis (Illumina), and Chain termination sequencing (Sanger sequencing).

In a more particular embodiment, optionally in combination with any embodiments above or below, mtDNA genetic variants are detected with specific probes comprising sequences complementary to any of nucleotide possibilities of each of the genetic variants.

Thus, present invention also relates to oligonucleotides complementary to the D-loop region wherein the nucleotide changes resulting in the variations of the present invention are located. Thus, they hybridize with a fragment of the nucleotide sequence carrying at least one of the mtDNA genetic variants. These oligonucleotides thus act as probes that allow detecting the nucleotide changes, in particular if there is a change G→A at position 16145 of rCRS, and/or a change T→C at position 16311 of rCRS, and/or a change T→C at position 72 of rCRS, and/or a change A→G at position 73 of rCRS A, and/or a change T→C at position 16356 of rCRS.

In yet another more particular embodiment of the first aspect, mtDNA genetic variants are determined by amplification of whole mtDNA or a part thereof with a set of primers selected from one or more of the following combinations of primers: Forward primer 5'-ATACACCAGTCTTGTAAACC-3' (SEQ ID NO: 1), reverse primer 5'-TTGAGGAGGTAAGCTACATA-3' (SEQ ID NO: 2);
Forward primers 5'-ATACACCAGTCTTGTAAACC-3' (SEQ ID NO: 1) and 5'-GAACTGTATCCGACATCTGG-3' (SEQ ID NO: 3); reverse primers 5'-GGTTAATAGGGTGATAGACC-3' (SEQ ID NO: 4) and 5'-GGGGTTTGGTGGAAATTTTT-3' (SEQ ID NO: 5)

In a more particular embodiment, the primers are those defined by forward primer 5'-CACCCATCAACAACCGCTATG-3' (SEQ ID NO: 7) and reverse primer 5'-GGATGAGGCAGGAATCAAAGAC-3'(SEQ ID NO: 8), and that allow amplification of a fragment containing the genetic variants of interest in the D-loop sequence of rCRS.

As indicated above, evaluation of clinical parameters (or classical cardiovascular risk factors) in combination with mtDNA mutations and/or chromosomic DNA mutations do also improve AUC in ROC analysis. Therefore, in another particular embodiment of the first aspect, the method further comprises considering one or more of the clinical parameters and/or features of the subject selected from hypercholesterolemia, hypertension, age, gender, systolic blood pressure, diastolic blood pressure, total-cholesterol levels, LDL-cholesterol levels, HDL-cholesterol, smoking habit, diabetes status, body mass index (BMI), triglyceride levels, hypolipemic drug consumption, blood pressure drug consumption, hypoglycaemic drug consumption and combinations thereof. In a more particular embodiment, the clinical parameter is selected from hypercholesterolemia, hypertension and combinations thereof.

In the context of the present invention "clinical parameter" or "clinical variable" or "clinical variable risk factor" (used interchangeably) relate to certain physical and/or biochemical features of the subject. For "features of the subject" is to be encompassed the age, the gender, the BMI, certain habits (i.e. smoking and medication).

As will be indicated below, all risk scores were obtained by conditional logistic regression with exception of nDNA-GRS, which determination is already disclosed in literature (see Lluís-Ganella et al., Atherosclerosis- 2012 (supra)). Briefly, for nDNA-GRS coronary risk was estimated using standard 10-year Framingham risk function and the REGICOR function, which is an adaptation of the former that has been validated and calibrated for Spanish population. Both functions included age, sex, systolic and diastolic blood pressure, total cholesterol level, HDL cholesterol level, smoking status, diabetes status and the GRS. Risk was computed using a particular formula disclosed by authors. Predictive capacity (capability to predict risk of CVD) of all risk scores were performed using ROC curves and DeLong test (DeLong et al. "Comparing the areas under two or more correlated receiver operating characteristic curves: a nonparametric approach", Biometrics.-1988, vol. no. 44(3), pp:837-45) was used for AUC comparison.

As indicated in examples below and with the large cohort of studied subjects, determining variants in mtDNA gave an AUC of 0.611 (0.567-0.655 with confidence interval CI of 95%). When corrected with the clinical variables hypertension and/or hypercholesterolemia, AUC value was of 0.739 (0.700-0.778, 95% CI) or of 0.764 in a sample cohort of different size, meanwhile AUC of the clinical variables was of 0.698 (0.658-0.737, 95% CI), being AUC 0.739 higher than 0.698 (p=0.0006) in a meaningful way. In addition, if the genetic risk score determined by means of nDNA variants (nDNA-GRS) was added, AUC raised to 0.774.

It was further calculated a risk score considering the relevant clinical variables hypertension and hypercholesterolemia together with the same GRS with variants in nDNA, which concluded with an AUC of 0.722.

P-value of AUC of 0.722 (score with nDNA GRS-and clinical variables) in relation with AUC of 0.774 (score with nDNA GRS-, mtDNA variants and clinical variables) was of 0.0005, indicating that relevant information was achieved with the combination of all the interrogated risk factors.

Minimal variations of AUC are not to be undervalued, since they imply the correct classification among subjects prone to display serious CVD.

Among the possible isolated sample of the tested subject, in a particular embodiment of the first aspect, the isolated sample is selected from blood (whole blood, serum or plasma), saliva, mucosa smear, mouth smear, urine, semen, tissue biopsies, biological material in paraffin, hair, pathological anatomic material, meanwhile mitochondrial DNA can be analysed from this isolated sample. Indeed, for the isolation of DNA it is generally prosecuted by extracting whole DNA from cells (nDNA and mtDNA). Since several copies per cell of mtDNA are present, even with small amounts of sample and/or with samples of bad quality, the variants of interest can be determined.

In yet another particular embodiment, the subject is from a population selected from the group consisting of subjects of Occidental Eurasia, North-African subjects, and American subjects from Europe colonialism (i.e. with low American indigenous inheritance). In a more particular embodiment the subject is from occidental Eurasia, more in particular from Europe, and even more in particular from Spain.

Another aspect of the invention is a method of deciding or recommending whether to initiate a prophylactic and/or therapeutic regimen of a subject suspected at risk of suffering from a CVD, which method comprises:
a) detecting, in vitro from an isolated sample of a subject and according to the method disclosed in the first aspect, if one or more genetic variants are present in D-loop of mtDNA of SEQ ID NO: 6 (or rCRS), wherein the genetic variants are selected from one or more of 16145G>A, 16311T>C, 16356T>C, 72T>C and 73A>G, and determining a risk score of suffering from a CVD; and
b) if the subject is considered at risk of suffering from a CVD, a prophylactic and/or therapeutic regimen is recommended.

All particular embodiments detailed above for the first aspect of the invention do also apply to this other aspect, relating to the method of deciding or recommending whether to initiate a prophylactic and/or therapeutic regimen of a subject suspected at risk of suffering from a CVD.

It is also disclosed herewith an in vitro method of identifying, in a sample taken from a subject, the presence or absence of an adenine (A) at position 16145 at position of human mtDNA of rCRS, that is detecting mutation defined as m. 16145G>A, the method comprising determining the sequence of mtDNA, or at least of D-loop in rCRS.

It is also disclosed an in vitro method of identifying, in a sample taken from a subject, the presence or absence of an adenine (A) at position 16145 of human mtDNA of rCRS, the method comprising determining the nucleotide at position 16145 by means selected from the group consisting of genotype methods.

The in vitro methods of identifying, in a sample taken from a subject, the presence or absence of an adenine (A) at position 16145 at position of human mtDNA of rCRS, further includes determining the nucleotide at least in one position selected from 16311, 16356, 72, and 73 of rCRS. More in particular, identifying the presence or absence of a cytosine at position 16311 of rCRS, and/or identifying the presence or absence of a cytosine (C) at position 72 of rCRS, and/or identifying the presence or absence of a guanine (G) at position 73 of rCRS, and/or identifying the presence or absence of a cytosine (C) at position 16356.

Also disclosed herewith are new oligonucleotides, which are probes complementary to the D-loop region wherein the nucleotide change resulting in the variation G→A at position 16145 of rCRS takes place. They have a nucleotide length from 10 to 50 nucleotides and, optionally, they comprise either in their 5'-end or 3'-end a marker (fluorescent marker, radioisotope marker) for the detection of the genetic variant.

In vitro methods of the invention according to any of the first and further aspects, as well as their particular embodiments, are in another particular embodiment carried out by means of kits or reagents, optionally in kits of parts, for detecting presence or absence of at least genetic variants in mtDNA, in particular any one or all of the five variants m.72T>C, m.73A>G, m.16145G>A and m.16311T>C, and m.16356T>C. Examples of reagents include specific probes and/or primers for amplifying the whole or a part of human mtDNA.

Indeed, present description also discloses an in vitro method for evaluating the risk of suffering from a CVD, the method comprising determining one or more variants in mtDNA or in a fragment thereof, in particular in D-loop of mtDNA, from an isolated sample of a subject, and further comprising determining in the isolated sample if one or more of nDNA variants are present; and/or considering one or more of the clinical parameters and/or features of the subject selected from hypercholesterolemia, hypertension, age, gender, systolic blood pressure, diastolic blood pressure, total-cholesterol levels, LDL-cholesterol levels, HDL-cholesterol, smoking habit, diabetes status, body mass index (BMI), triglyceride levels, hypolipemic drug consumption, blood pressure drug consumption, hypoglycaemic drug consumption and combinations thereof.

According to inventor's knowledge this is the first time it has been proposed the combination of either clinical variables associated with CVD or nDNA variants also associated with CVD, with any variant in mtDNA of an isolated sample of a subject. Data provided herewith demonstrate that such combinations of risk factors lead to accurate evaluation of risk of CVD, allowing higher discrimination (as indicated by AUC values) in comparison with the factors considered independently.

As indicated, more particularly genetic variants in mtDNA comprise one or more of 16145G>A, 16311T>C, 16356T>C, 72T>C and73A>G. Also in particular, CVD are selected from MI and stroke.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Example

Example 1. Control region mitochondrial DNA mutations in CVD Next paragraphs illustrate the detection of the independent risk score factors for CVD of the invention.

### 1.1 Materials and methods

In this study, data from 730 subjects (154 individuals with stroke history, 211 individuals with MI history and their corresponding control individuals -matched for age, gender and geographic origin-), were used. Samples come from the Cardiovascular Disease Risk Study of Castile and Leon (Vega et al. "Design of a population-based study of cardiovascular risk in Castile and Leon (Spain) through primary care teams". Gac Sanit 2007, vol no. 21, pp.: 84-87), whose design and analysis have already been described by Umbria et al. "Involvement of mitochondrial haplogroups in myocardial infarction and stroke: A case-control study in Castile and Leon (Spain) population", Mitochondrion 2017, https://doi.org/10.1016/j.mito.2017.12.004. In brief, possible association between mtDNA haplogroups and two CVDs (stroke and MI) was evaluated and classical cardiovascular (CV) risk factors. For each individual, information about history of hypertension (≥140/90 mmHg), history of diabetes, history of hypercholesterolemia (>200 mg/dl), cigarette consumption (smokers, former smokers and non-smokers), presence of overweight or obesity (body mass index ≥25 kg/m2), presence of high abdominal perimeter in risk range (risk: ≥80 cm for women and ≥94 cm for men) and presence of high levels of triglycerides (≥170 mg/dl) was obtained.

### 1.2 MtDNA sequence analysis and heteroplasmy authentication

The mtDNA sequences used in the present study were previously obtained by Umbria et al. (*supra*), although, they were strictly used to classify samples into mtDNA haplogroups. Briefly, for each sample, the control region of the mtDNA was amplified between positons 15907 and 580 using primers L15907, Forward primer 5'-ATACACCAGTCTTGTAAACC-3' (SEQ ID NO: 1), and H580, reverse primer 5'-TTGAGGAGGTAAGCTACATA-3' (SEQ ID NO: 2). PCR was performed in a final volume of 50µl using standard conditions of BIOTAQ polymerase (Bioline, Taunton, USA). The PCR program consisted in an initialization step of 5 min at 94 °C, 35 PCR cycles (1 min at 94 °C, 30 s at 58 °C, 50 s at 72 °C) and a final step of 5 min at 72 °C. PCR products were purified using ExoSAP-IT (Affymetrix, Santa Clara, California) and were further sequenced using two forward primers, L15907, 5'-ATACACCAGTCTTGTAAACC-3' (SEQ ID NO: 1) and L16485, 5'-GAACTGTATCCGACATCTGG-3' (SEQ ID NO: 3) and two reverse primers, H1_8, 5'-GGTTAATAGGGTGATAGACC-3' (SEQ ID NO: 4) and H2_288, 5'-GGGGTTTGGTGGAAATTTTT-3' (SEQ ID NO: 5). Sequence reactions were carried out using Big Dye® Terminator v.3.1 Cycle Sequencing Kit (Applied Biosystems, Foster City, USA) according to the manufacturer's specifications. Purification of DNA sequencing reactions was performed with BigDye XTerminator® Purification Kit (Applied Biosystems, Foster City, USA).

In the present study, sequences were reassessed and analysed at the nucleotide level to identify not only fixed variants but also variants in heteroplasmy. The alignment in relation to the revised Cambridge Reference Sequence (rCRS) (see Andrews, et al., "Reanalysis and revision of the Cambridge reference sequence for human mitochondrial DNA" Nature genetics,1999, vol. no. 23, p.147) and the heteroplasmy detection were performed using the SeqScape 2.5 software (Applied Byosistems, Foster City, USA) considering a value of 5% in the Mixed Base Identification option. Only sequences with satisfactory peak intensity and without background/noise were considered. In this context, some samples were amplified and sequenced several times (using the same methodology described in Umbria et al. (*supra*) to obtain accurate sequences to heteroplasmy detection. Moreover, additional analyses were performed in order to authenticate such heteroplasmies.

The authentication of mtDNA heteroplasmy was performed following a similar strategy to that used by Santos et al. "Understanding differences between phylogenetic and pedigree-derived mtDNA variant rate: a model using families from the Azores Islands (Portugal)", Molecular biology and evolution, 2005, vol. no. 22, pp.:1490-1505).
1. PCR amplification and sequencing of the control region of the mtDNA.
2. To authenticate the results for samples presenting heteroplasmy in step 1, a second PCR amplification and sequencing were performed.
3. In addition, to exclude a possible contamination of the samples, an analysis of Short Tandem Repeat (STR) DNA profiling was carried out employing AmpFISTR® Identifiler® PCR Amplification Kit (Applied Biosystems, Foster City, USA) following the manufacturer's protocol.

Thus, point heteroplasmic positions were accepted if they appeared in all the validation steps and no evidence of sample contamination was detected. Levels of heteroplasmy were determined using the height of peaks in the electropherograms. To calculate the average heteroplasmic levels, the results obtained for at least two sequence reads of each heteroplasmic position were used.

### 1.3 Data Analysis

### Statistical analyses:

To compare differences in the CR profile between cases and controls in both stroke and MI, all fixed mtDNA variants and all heteroplasmic variants were compiled into a matrix considering the cases and controls analysed for each disease. The set of fixed mtDNA variants detected in cases and controls (present in a minimum of two individuals) were compared using McNemar's test. To adjust the association analysis for the potential confounding effect of clinical variables (CV) risk factors detected previously in Umbria et al. (*supra*), a conditional logistic regression analysis (forward stepwise model) was applied. Hypercholesterolemia was considered a CV risk factor with a potential confounding effect for stroke, while both hypertension and hypercholesteroplemia were considered for MI samples. Therefore, Odds Ratios (ORs) and their 95% Confidence Intervals (CIs) were calculated adjusting for the effect of these risk factors in each disease. McNemar's test was applied to compare the presence or absence of point (PH) and length (LH) heteroplasmy, and a logistic regression analysis was used to correct for the effect of CV risk factors above mentioned. Finally, the mtDNA variants were analysed to evaluated if they were in positions defined by haplogroups (previously examined by Umbria et al. (*supra*)), or acts as an independent genetic factor. Statistical analyses were performed using IBM SPSS ver. 22.0 (SPSS Inc.). All differences were considered significant at p<0.05.

### Hits in the phylogeny, population database and Conservation Index (CI):

The stability of fixed mtDNA variants and point heteroplasmic position were analysed as previously detailed by Ramos et al "Frequency and pattern of heteroplasmy in the complete human mitochondrial genome. PloS one, 2003, 8, e74636). The number of hits in the phylogeny for each position was compiled from the updated mtDNA phylogeny - mit. Tree build 1734 - and from Soares et al., "Correcting for Purifying Selection: An Improved Human Mitochondrial Molecular Clock", The American Journal of Human Genetics, 2009, vol no. 84, pp.: 740-759). From these data, it has been possible to calculate the probability of variant as the ratio between the observed and the total number of hits. An mtDNA position was considered a hotspot if the variant probability was ten time higher that the expected mean value. In order to calculate the frequency of each variant for a particular nucleotide position, a database of 3880 mtDNA complete sequences was used. Sequences were aligned using Clustal W and formatted for further frequency analyses using the SPSS software. Sequences were analyzed using the same method previously mentioned.

### Structure prediction:

Secondary structures were performed to understand the structural impact of different variants found. The secondary structures for each position was generated from sequences (A-M) identified by Pereira et al., "Evidence for variable selective pressures at a large secondary structure of the human mitochondrial DNA control region", Mol Biol Evol, 2008, vol no. 25, pp.: 2759-2770. All sequences were submitted to the RNAfold web server (http://rna.tbi.univie.ac.at/cgi-bin/RNAWebSuite/RNAfold.cgi) using default parameters for DNA secondary structures calculations. The minimum free energy prediction and base pair probabilities were used to estimate the implication in the molecule.

### 1.4. Results

### Analysis of fixed and heteroplasmic mtDNA variants with stroke:

Frequencies of fixed variants found are showed in Table 1. The percentages of m.16145G>A and m.16311T>C were overrepresented in stroke cases (5.2% and 18.2%, respectively) than controls (1.9% and 9.7%, respectively). After correction for the effect of CV risk factors with significant differences between stroke cases and controls (hypercholesterolemia), significant association were still observed in m.16145G>A (conditional logistic regression: p=0.038; OR= 4.407, 95% CI [1.086-17.883]) and m.16311T>C (conditional logistic regression: p=0.018; OR= 2.417, 95% CI [1.165-5.016]), emerging as a possible genetic risks factors for stroke.

**Table 1.Complete results of stroke fixed mtDNA variants analyzed**

| | **Position 16145G>A** | **Position 16311T>C** |
|---|---|---|
| Controls: n=154 (%)/ Cases: n=154 (%) | 3 (1.9) / 8 (5.2) | 15 (9.7) / 28 (18.2) |

| Logistic Regression¹ | | |
|---|---|---|
| p-value | 0.038 | 0.018 |
| OR [95% CI] | 4.407 [1.086-17.883] | 2.417 [1.165-5.016] |

| Stability analysis | | |
|---|---|---|
| CRS | G | T |
| Distribution in population database | G:97.2; A:2.8; GAP:0.02 | T:76.9; C:23.0; Y:0.02; GAP:0.02 |
| No. Hits phylogeny (PhyloTree.org) | 37 | 137 |
| No. Hits Soares et al.³⁶ | 22 | 120 |
| Probability of mutation | 0,00205935 | 0,01123280 |
| Nucleotide Conservation Index (%) | A:56.3; G:20.8; T:14.6; C:8.3 | T:58.3; C:33.3; G:4.2; A:2.1; GAP:2.1 |

| | | |
|---|---|---|
| ¹Conditional regression model was performed considering significant covariates for stroke (hypercholesterolemia and mtDNA mutations) | | |

Stability analyses were performed to predict the impact of these variants. Several measures as the number of hits in the mtDNA phylogeny, the probability of variant, the frequency in the population database and the conservation index (CI) at nucleotide level, were calculated, and results are showed in former Table 1. The results obtained revealed m.16145G>Aand m.16311T>C as non-stable position since they present a minimum of 37 hits in the phylogeny, a high probability of mutation, a high frequency of the variant in the population database (here denoted by minor allele frequency [MAF] >5%) detected on m.16311T>C or low-frequency (MAF 1-5%) in m.16145G>A and a maximum nucleotide CI of 58% (Table 1). To infer about the impact of m.16145G>A and m.16311T>C on the stability of secondary structures of the mtDNA, a prediction of different structures with the wild type (rCRS) and mutant variant was performed. It seems that m.16311T>C implies a conformational rearrangement, causing a stability reduction of the region. No structural or thermodynamic differences were found for the m.16145G>A.

In relation with heteroplasmies, no meaningful differences were detected between controls and stroke cases in analysed population.

### Analysis of fixed mtDNA mutations with MI:

MtDNA positions studied for MI cases and controls and the frequencies of fixed mutations found are reported in Table 2. The m.72T>C, m.73A>G and m.16356T>C were more frequent in MI controls (12.3%, 49.3% and 3.3%, respectively) than cases (7.6%, 38.9% and 1.4%, respectively). When corrected for the effect of CV risk factors with significant differences between MI cases and control (hypertension and hypercholesterolemia29), significant association was observed in these tree mutations (m.72T>C: conditional logistic regression: p=0.001; OR= 0.041, 95% CI [0.006-0.290], m.73A>G: conditional logistic regression: p=0.009; OR= 0.009, 95% CI [0.307-0.843] and m.16356T>C: conditional logistic regression: p=0.016; OR= 0.091, 95% CI [0.013-0.639]), emerging as a possible protective genetic factors for MI.

In order to predict the impact of these variants, several measures were calculated to analyze the stability of each position, and results are showed in Table 2. The results obtained revealed that m.72T>C, m.73A>G and m.16356T>C as a non-stable positions since they present a minimum of 9 hits in the phylogeny, a high probability of variant, a high frequency of the variant in the population database (MAF >5%) for m.73A>G and low-frequency (MAF 1-5%) for m.72T>C and m.16356T>C, and a maximum nucleotide CI of 79%. Using the proposed previously method to predict the impact of these three mutations on the stability of secondary structure of the mtDNA, it seems that m.72T>C, m.73A>G and m.16356T>C led to a folded structure with the same minimum free energy as the wild-type structure (rCRS), which means that these variants do not condition the stability of the region.

**Table 2: Complete results of myocardial infarction fixed mtDNA mutation analysed**

| | Position 72T>C | Position 73A>G | Position 16356T>C |
|---|---|---|---|
| Controls: n=211 (%) / Cases: n=211 (%) | 26 (12.3) / 16 (7.6) | 104 (49.3) / 82 (38.9) | 7 (3.32) / 3 (1.42) |
| Logistic Regression¹ | | | |
| p-value | 0.001 | 0.009 | 0.016 |
| OR [95% CI] | 0.041 [0.006-0.290] | 0.509 [0.307-0.843] | 0.091 [0.013-0.639] |
| Stability analysis | | | |
| CRS | T | A | T |
| Distribution in population database | T:97.4; C:2.4; G:0.1; GAP:0.1 | G:80.8; A:19.1; GAP:0.1; C:0.02 | T:97.5; C:2.5; GAP:0.02 |
| No. Hits phylogeny (PhyloTree.org) | 9 | 12 | 15 |
| No. Hits Soares et al.³⁶ | 6 | 11 | 19 |
| Probability of mutation | 0,00056164 | 0,00102967 | 0,00177853 |
| Nucleotide Conservation Index (%) | T:77.1; GAP:8.3; A:6.3; C:4.2; G:4.2 | A:41.7; C:35.4; G:22.9 | T:79.2; C:.12.5; A:6.3; GAP:2.1 |

| | | | |
|---|---|---|---|
| ¹Conditional regression model was performed considering significant covariates for MI (hypertension, hypercholesterolemia and mtDNA mutations) | | | |

In relation with heteroplasmies, no significative differences were detected between controls and MI cases in analysed population.

All these results allow concluding that significant differences were found in the two diseases when compared to controls, reporting the m.16145G>A and m.16311T>C as a potential genetic risk factors for stroke, and m.72T>C, m.73A>G and m.16356T>C as a possible beneficial genetic factors for MI.

The D-loop variants do not act directly on the ETC affecting mitochondria bioenergetics or ROS generation; and without being bound to any theory they may have an important effect on the genotype by altering mtDNA gene expression. The CR contains the main regulatory sequences for replication initiation and transcription. Unfortunately, the potential role of the remaining areas of the CR is still unknown even considering the exceptional economy of organization of the mtDNA.

Transitions 16145G>A and 16311T>C seem to have a pathogenic role in stroke. The analysis of distribution of these variants clearly showed that they are located in many different haplogroups and consequently these mutations act as haplogroup-independent risk factors. The m. 16145G>A is located between MT-TAS sequence (nt. 16157-16172 of rCRS) and MT-TAS2 sequence (nt. 16081-16138 of rCRS). According to the classic strand-asynchronous mechanism, recent studies demonstrated that the 5'end of the D-loop is capable of forming secondary structures, which act as a recognition site to molecules involved in the premature arrest of H strand elongation. The biological importance of this region was confirmed by some scientists who also observed multiple tumor specific mutations in the pre-TAS region. These observations suggest that variants arising near to this conserved motive might be responsible of the alterations in mtDNA replication and transcription. In the same line, m.16311T>C has been found to be significantly associated with certain types of cancer (see Chen et al., "Extensive somatic mitochondrial mutations in primary prostate cancer using laser capture microdissection", Cancer research, 2002, vol.no. 62, pp.: 6470-6474). This variant is located between the control elements Mt5 sequence (nt. 16194-16208 of rCRS) and the Mt3l sequence (nt. 16499-16506 of rCRS). In this case, data from current invention showed that m.16311T>C may imply a reduction in the stability of secondary structure of this region, which would affect in the binding grade to mtDNA transcription factors, ultimately affecting on the intensity of transcription regulation. In both cases, these findings strongly suggest that mtDNA CR dysfunction may cause a decrease on the mtDNA copy number, which could affect the efficiency of ETC, lowering the ATP:ADP ratio and increasing ROS production, contributing in stroke development.

Concerning MI, the results showed that m.72T>C, m.73A>G and m.16356T>C act as a beneficial factor for MI. Although a high percentage of individuals with these variants belonged to the haplogroups HV0, H or U, which have been shown to may have higher oxidative damage, the distribution of positions 72, 73 and 16356 in tested samples was independent of these haplogroups. Although m.72T>C, m.73A>G and m.16356T>C have been previously related to certain types of cancer (see Kirches et al., "High frequency of mitochondrial DNA mutations in glioblastoma multiform identified by direct sequence comparison to blood samples", International journal of cancer, 2001, vol. no. 93, pp.: 534-538), many studies consider that they are recurrent variants common in humans.

Even though the most deleterious variants are removed by natural selection, a wide range of milder bioenergetic alterations are introduced in certain populations. Some of these variants as m.72T>C, m.73A>G and m.16356T>C could be advantageous and seen as a way to facilitate survival in specific environments. In contrary, other variants, such as m.16145G>A and m.16311T>C escape of intraovarian selection and could cause significant mitochondrial defects and stroke develop. Much of the progress in linkage disequilibrium mapping of complex diseases has been made using the major assumptions of the CDCV hypothesis, that is, that common alleles cause common diseases.

All these genetic variants detected as indicated above with methodology of Umbria el at. (supra) are also detected with the new set of primers: F16069_D-loop: 5'-CACCCATCAACAACCGCTATG-3' (SEQ ID NO: 7) and R151_D-loop: 5'-GGATGAGGCAGGAATCAAAGAC-3' 8SEQ ID NO: 8). With this set of primers a PCR fragment with a size og 652 nucleotides is obtained. This allows the genotyping of the positions of interest using PCR of the D-loop region followed by automated Sanger sequencing.

PCR size: 652
Temp. Annealing: 58°C

The PCR was performed in a final volume of 50µl using standard conditions of BIOTAQ polymerase (Bioline, Taunton, USA). The PCR program consisted in an initialization step of 5 min at 94 °C, 35 PCR cycles (1 min at 94 °C, 30 s at 58 °C, 50 s at 72 °C) and a final step of 5 min at 72 °C. The amplification of each sample was verified through electrophoresis with 1.5 % agarose. PCR products were purified using ExoSAP-IT (Affymetrix, Santa Clara, California) and were further sequenced using forward (F16069_D-loop) and reverse primer (R151_D-loop). Sequence reactions were carried out using Big Dye® Terminator v.3.1 Cycle Sequencing Kit (Applied Biosystems, Foster City, USA) according to the manufacturer's specifications. Purification of DNA sequencing reactions was performed with BigDye XTerminator® Purification Kit (Applied Biosystems, Foster City, USA).

In parallel, risk scores with the combination of several risk factors (including classical cardiovascular risk factors, or clinical parameters or clinical variables) were calculated for this cohort including 211 cases of MI with the corresponding 211 controls; and 154 cases of stroke with corresponding 154 controls.

A clinical risk score with main clinical variables differing controls in a meaningful way from events was first calculated (hypertension and hypercholesterolemia). This score was used to evaluate the improve that was added to a GRS of mtDNA. GRS of mtDNA was obtained from the 5 positions of mtDNA, which were related to MI or stroke in the analysed set of samples.

As will be indicated below, all risk scores were obtained by conditional logistic regression Predictive capability of all risk scores were performed using ROC curves, and Delong test (see DeLong et al., supra) was used for AUC comparison. Results are depicted in Table 3 below.

**Table 3. Predictive capacity (AUC) of variants in D-loop mtDNA**

| 304CTL/304CASES | AUC | 95%CI | p (Mi.vs.Mj) |
|---|---|---|---|
| M1:Clinic vars | 0.698 | 0.658-0.737 | 0.0006 |
| M2:Clinic+mtDNA | 0.739 | 0.700-0.778 | |
| M3: Only mtDNA | 0.611 | 0.567-0.655 | |
| | | | |
| | | | |
| M1:Clinic vars | HYPERTENSION | | |
| | HYPERCHOLESTEROLEM IA | | |
| | | | |
| M2:Clinic+Genetic | HYPERTENSION | | |
| | HYPERCHOLESTEROLEM IA | | |
| | 72C | | |
| | 73G | | |
| | 16356C | | |
| | 16145A | | |
| | 16311C | | |

Reference score M1 (clinical variables) has an AUC of 0.698 that, as indicated by its CI is higher than 0.5 in a significative mode (being 0.5 the value indicating a null discrimination capacity). When mtDNA score was added to the clinical variables, new score (M2) was of 0.739, being higher than 0.698 (p=0.0006) in a significative manner.

### Example 2. Predictive capacity of different risk scores for CVD

To test if these mtDNA variants determined in Example 1 modestly improved risk prediction to CVD beyond traditional risk factors and results obtained only from nuclear GRS, the magnitude of the association between an individual GRS based on the set of these mitochondrial genetic variants, nuclear genetic variants and the presence of CVD was determined.

First, it was assessed, separately, the capability of nuclear and mitochondrial GRS, to improve the risk prediction of CVDs events in the same Spain cohort used in Example 1. Secondly, it was tested whether the addition of mitochondrial variants to nuclear GRS already established and tested in other populations, improved predictive risk capacity.

### 2.1 Materials and methods

As in Example 1, the cases and controls analysed in this study were selected from the Cardiovascular Disease Risk Study of Castile and Leon41, a cohort study performed in Castile and Leon (centre-north region of Spain), where a random sample from general population underwent a health examination in relation with cardiovascular risk factors in 2004. These individuals were followed up with the same methodology in 2009 and 2014. For each individual, it was also obtained information about age (categories ≤44, [45-49], [50-54], [55-59], [60-64], ≥65 years), gender, geographic origin (North, Central and South regions of Castile and Leon), history of hypertension, history of diabetes, history of hypercholesterolemia, cigarette consumption (smokers, former smokers and non-smokers), presence of overweight or obesity (body mass index ≥25 kg/m2), presence of high abdominal perimeter in risk range (risk: ≥80 cm for women and ≥94 cm for men) and presence of high levels of triglycerides (≥170 mg/dl).

To determine the magnitude of the association between an individual GRS using mtDNA genetic variants and the presence of CVD, a total of 301 cases with clinical history of CVD and 221 controls were included in this study. For each sample, the control region of mtDNA were analysed at the nucleotide level to identify fixed and heteroplasmy mutations. These results have been previously disclosed in Example 1 for a total of 501 samples (280 cases and 221 controls), whereas for the remaining 21 individuals, these analyses were performed following the same methodology explained in Umbria et al. (supra).

### 2.2. Selection of genetic variants, genotyping and multi-locus risk score generation

mtDNA genetic variants to assess the association between mitochondrial GRS and incident CVDs were selected from data previously analysed in Example 1. The variants selected were m.16145G>A and m.16311 T>C as a potential genetic risk factors for stroke and m.72T>C, m.73A>G and m.16356T>C that could act as a possible beneficial genetic factors for MI.

The selected variants to generate the nuclear GRS (nDNA GRS) are described by Luís-Granella et al. (supra). In brief, the 11 selected genetic variants were associated with CVDs (p ≤ 1 x 10⁻⁶) but no with CVRFs (age, gender, origin, cigarette consumption, hypertension, , diabetes, hypercholesterolemia, overweight or obesity, abdominal perimeter and triglycerides), and are not related to each other by linkage disequilibrium (r² > 0.3). The list included: rs10455872 in LPA; rs10507391, rs17222842 and rs9315051 in ALOX5AP; rs12526453 in PHACTR1; rs1333049 near CDKN2A/2B; rs17465637 in MIA3; rs501120 in CXCL12; rs6725887 in WDR12; rs9818870 in MRAS and rs9982601 near SLC5A3, MRPS6, KCNE2.

### 2.3. Estimation of individual risk scores

In order to assess the capability of nuclear and mitochondrial genetic information to improve risk prediction we first developed an individual risk score based on clinical variables, considering those variables significantly different (p<0.05) between cases and controls. Then, it was added the nuclear GRS described in Lluís-Ganella et al (supra) as a new variable to the clinical risk score, generating a new risk score with clinical and genetic information to explore the utility of this GRS in the study population. The same analysis was repeated with mitochondrial information reported in Example 1 to generate a third risk score that would allow us to compare the predictive capability of mitochondrial versus nuclear GRS. After this step, the mitochondrial variables were added to second risk score, developing a new risk score with clinical and genetic information (nuclear GRS and mitochondrial variables).

Each new risk score was compared against the previous one, to assess if the addition of new information was relevant, in terms of predictive capability. All risk scores were implemented using multivariate conditional logistic regression models.

### 2.4. Statistical analysis

Baseline demographic and clinical variables of different groups of individuals was compared through the χ2 test for categorical variables. All differences were considered significant at p<0.05.

Multivariate conditional logistic regression models were used to develop the different risk scores. Predictive capacity of risk scores was evaluated using the area under the receiver operating characteristic (ROC) curve (AUC, larger values indicating better discrimination). Comparison of AUCs was performed with the DeLong's test for paired data (Delong et al., *supra*). Statistical analyses were performed using R statistical software, version 3.1.3 (R Development Core Team, 2015).

### 3.5. Results

Table 4 shows a summary of the sociodemographic, biochemical and clinical characteristics of selected cases and controls. Hypertension and hypercholesterolemia were significantly higher in cases than in controls [(hypertension: 50.2% vs. 30.5%, respectively; p<0.001, χ² test) and (hypercholesterolemia: 38.1% *vs.* 19.2%, respectively; p<0.001, χ² test)] (Table 4). The frequency of individuals with diabetes, overweight or obesity, abdominal perimeter in risk range, and high triglycerides, was similar in cases and controls.

The frequency for each mitochondrial and nuclear variant that were included in the analyses for cases and controls are showed in Table 5. For mitochondrial risk variants, higher frequencies were observed in cases. By contrast, beneficial variants were more frequents in controls. The distribution of the risk allele for each of the nuclear variants analysed is similar between cases and controls (Table 5).

The risk score developed only with clinical variables included hypertension and hypercholesterolemia as the clinical relevant predictors (Table 6). The AUC for this score was 0.697 (95%CI: 0.655-0.739). When the nDNA GRS described in Lluís-Ganella et al. (supra) was included as a new predictor jointly with the clinical variables the new score increased its AUC to 0.722 (95%CI: 0.677-0.768), showing a statistically significant improvement in the predictive capacity, AUCs 0.722 vs 0.697 (p=0.032). The same analysis was carried out including the mitochondrial GRS with risk score based on clinical variables. In this case, the estimated risk increased its AUC to 0.764 (95%CI: 0.722-0.806), also showing a statistically significant improvement in the predictive capacity, (Table 6). These results indicated that the utilization of mitochondrial variants also improves the risk prediction to CVD predictive capacity beyond CVRFs by a similar percentage or slightly higher to that obtained by the nuclear GRS.

From the previous risk score it was developed a new one by adding jointly the nDNA GRS and the mtDNA variables reported in Example 1 (Table 6). The AUC obtained with this risk score developed with clinical variables, the nDNA GRS and the mtDNA variables was 0.774, improving significantly the predictive capability of the previous risk score (0.774 vs 0.722, p=0.001). In this score, two of the mtDNA variables (m.16311T>C and m.73A>G) could be excluded from the model without any significant effect in its predictive performance (AUC=0.772) in this population.

All these data allow concluding that when mtDNA variables (that is, mt DNA genetic variants) are also considered in the combined score defined by nDNA GRS + conventional /clinical variables risk score, a significant improvement in the risk prediction was obtained. To date, there are many studies utilizing multiple variants previously associated with different CVDs in hope of improving the predictive risk. But, to the best of inventor's knowledge, the present study is the first to demonstrate that utilization of a GRS based on mtDNA variants did significantly improve short-term (10-years) CVD prediction beyond the information provided by clinical variants as risk factor (CVRFs) alone. Therefore, these findings open the doors for new studies to include mitochondrial variants in genetic risk scores (GRS).

**Table 4. Sociodemographic, biochemical and clinical characteristics of cases and controls**

| | **Controls** | **Cases CVD** | |
|---|---|---|---|
| | **n=221 (%)** | **n=301 (%)** | **P-value¹** |
| Age (years) | | | |
| ≤44 | 4 (0.8) | 6 (1.1) | |
| 45-49 | 14 (2.7) | 13 (2.5) | |
| 50-54 | 5 (1) | 12 (2.3) | 0.618 |
| 55-59 | 19 (3.6) | 18 (3.4) | |
| 60-64 | 24 (4.6) | 34 (6.5) | |
| ≥65 | 155 (29.7) | 218(41.8) | |
| Male/Female | 129 (24.7)/ 92 (17.6) | 189 (36.2)/ 112 (21.5) | 0.307 |

| Geographic origin | | | |
|---|---|---|---|
| North | 64 (12.3) | 79 (15.1) | |
| Central | 92 (17.6) | 124 (23.8) | 0.687 |
| South | 65 (12.5) | 98 (18.8) | |

| Cigarette consumption | | | |
|---|---|---|---|
| Non-smoking | 126 (24.1) | 151 (28.9) | |
| Former smoker | 64 (12.3) | 111 (21.3) | 0.164 |
| Smoker | 31 (5.9) | 39 (7.5) | |
| Hypertension | 159 (30.5) | 262 (50.2) | <0.001 |
| Diabetes | 41 (7.9) | 68 (13) | 0.262 |
| Hypercholesterolemia | 100 (19.2) | 199 (38.1) | <0.001 |
| Overweight or obesity (≥25kg/m2) | 170 (32.6) | 221 (42.3) | 0.362 |
| High abdominal perimeter (>80 or >94 cm) | 173 (33.1) | 218 (41.8) | 0.127 |
| Triglycerides (≥170mg/dl) | 31 (5.9) | 37 (7.1) | 0.561 |

| | | | |
|---|---|---|---|
| ¹P-value of an unpaired chi-squared test for qualitative data | | | |

**Table 5. MtDNA and nDNA variants frequencies (%) of cases and controls.**

| Variants | DNA | Number of individuals (%) | |
|---|---|---|---|
| | | Controls (n=221) | Cases (n=301) |
| m.16145G>A | mitochondrial | 8 (3.6) | 13 (4.3) |
| m.16311T>C | mitochondrial | 29 (13.1) | 46 (15.3) |
| m.72T>C | mitochondrial | 28 (12.7) | 18 (6) |
| m.73A>G | mitochondrial | 98 (44.3) | 127 (42.2) |
| m.16356T>C | mitochondrial | 8 (3.6) | 4 (1.3) |
| rs10455872 | nuclear | 26 (11.8) | 56 (18.6) |
| rs10507391 | nuclear | 191 (86.4) | 250 (83.1) |
| rs12526453 | nuclear | 188 (85.1) | 264 (87.7) |
| rs1333049 | nuclear | 168 (76) | 241 (80.1) |
| rs17222842 | nuclear | 30 (13.6) | 40 (13.3) |
| rs17465637 | nuclear | 199 (90) | 280 (93) |
| rs501120 | nuclear | 216 (97.7) | 296 (98.3) |
| rs6725887 | nuclear | 57 (25.8) | 91 (30.2) |
| rs9818870 | nuclear | 36 (16.3) | 49 (16.3) |
| rs9982601 | nuclear | 57 (25.8) | 72 (23.9) |
| rs9315051 | nuclear | 40 (18.1) | 62 (20.6) |

**Table 6. Adjusted models for calculation of different risk scores and comparison of AUCs. Predictive capacity of risk scores.**

| Risk score 1 | | Risk score 2 | | Risk score 3 | | Risk score 4 | |
|---|---|---|---|---|---|---|---|
| Var. | Pvalue | Var. | Pvalue | Var. | Pvalue | Var. | Pvalue |
| Hypert. | 0.0001 | Hypert. | 0.0001 | Hypert. | 0.0001 | Hypert. | <0.0001 |
| Hyperchol. | 0.0032 | Hyperchol. | 0.0018 | Hyperchol. | 0.0018 | Hyperchol. | 0.0022 |
| | | nDNA- | 0.0271 | m.16145G>A | 0.1023 | nDNA-GRS | 0.0705 |
| | | GRS | | m.16311T>C | 0.4054 | m.16145G>A | 0.1042 |
| | | | | m.72T>C | 0.0036 | m.16311T>C | 0.4592 |
| | | | | m.73A>G | 0.2834 | m.72T>C | 0.0060 |
| | | | | m.16356T>C | 0.0199 | m.73A>G | 0.3420 |
| | | | | | | m.16356T>C | 0.0271 |
| AUC1 0.697 | | AUC2 0.722 | | AUC3 0.764 | | AUC4 0.774 | |
| | P-value | | | | | | |
| AUC1 vs AUC2 | 0.0323 | | | | | | |
| AUC2 vs AUC3 | 0.0005 | | | | | | |

| | | | | | | | |
|---|---|---|---|---|---|---|---|
| Var.: Variable Hypert.: Hypertension Hyperchol.: Hypercholesterolemia | | | | | | | |

### Citation List

### Non Patent Literature

- Taverne et al.2013 "Reactive oxygen species and the cardiovascular system. Oxid Med Cell Longev 2013, 862423.
- DeLong et al. "Comparing the areas under two or more correlated receiver operating characteristic curves: a nonparametric approach", Biometrics.-1988, vol. no. 44(3), pp:837-45.
- Beaney et al., "How close are we to implementing a genetic risk score for coronary heart disease?", Expert Review of Molecular Diagnostics 2017, DOI: 10.1080/14737159.2017.1368388.
- Christiansen et al. "A genetic risk score predicts cardiovascular events in patients with stable coronary artery disease", International Journal of Cardiology 2017, http://dx.doi.org/10.1016/j.ijcard.2017.04.045.
- Hudson et al., "Recent Mitochondrial DNA Mutations Increase the Risk of Developing Common Late-Onset Human Diseases", PLoS Genet 10 2014, e1004369.
- Wang et al. "Cardiovascular Disease, Mitochondria, and Traditional Chinese Medicine". Evidence-Based Complementary and Alternative Medicine 2015, 7.
- Gawe 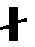 et al., "The G16319A substitution frequency in a hemorrhagic stroke", Annals of Indian Academy of Neurology 2008, vol. no. 11, pp.:154-158.
- Andrews, et al., "Reanalysis and revision of the Cambridge reference sequence for human mitochondrial DNA" Nature genetics,1999, vol. no. 23, p.147.
- Vega et al. "Design of a population-based study of cardiovascular risk in Castile and Leon (Spain) through primary care teams". Gac Sanit 2007, vol no. 21, pp.: 84-87.
- Umbria et al. "Involvement of mitochondrial haplogrups in myocardial infarction and stroke: A case-control study in Castile and Leon (Spain) population", Mitichondrion 2017, https://doi.org/10.1016/j.mito.2017.12.004.
- Santos et al. "Understanding differences between phylogenetic and pedigree-derived mtDNA variant rate: a model using families from the Azores Islands (Portugal)", Molecular biology and evolution, 2005, vol. no. 22, pp.:1490-1505.
- Ramos et al "Frequency and pattern of heteroplasmy in the complete human mitochondrial genome. PloS one, 2003, 8, e74636.
- Soares et al., "Correcting for Purifying Selection: An Improved Human Mitochondrial Molecular Clock", The American Journal of Human Genetics, 2009, vol no. 84, pp.: 740-759.
- Pereira et al., "Evidence for variable selective pressures at a large secondary structure of the human mitochondrial DNA control region", Mol Biol Evol, 2008, vol no. 25, pp.: 2759-2770.
- Chen et al., "Extensive somatic mitochondrial mutations in primary prostate cancer using laser capture microdissection", Cancer research, 2002, vol.no. 62, pp.: 6470-6474.
- Kirches et al., "High frequency of mitochondrial DNA mutations in glioblastoma multiform identified by direct sequence comparison to blood samples", International journal of cancer, 2001, vol. no. 93, pp.: 534-538.

## Claims

1. An *in vitro* method for evaluating the risk of suffering from a cardiovascular disease, the method comprising determining in an isolated sample of a subject, if one or more genetic variants are present in D-loop sequence of mitochondrial DNA of SEQ ID NO: 6, wherein the genetic variants are selected from one or more of 16145G>A, 16311T>C, 16356T>C 72T>C and 73A>G.

2. The *in vitro* method according to claim 1, wherein the cardiovascular disease is selected from stroke, myocardial infarct, angina pectoris, coronary revascularization, acute coronary syndrome, peripheral arterial disease, and combinations thereof.

3. The *in vitro* method according to any one of claims 1-2, wherein the cardiovascular disease is selected from stroke, myocardial infarct and combinations thereof.

4. The *in vitro* method according to any one of claims 1-3, wherein if at least one of 16145G>A, 16311T>C genetic variants is detected, it is concluded that the subject comprises a genetic variant increasing probability of suffering from at least stroke.

5. The *in vitro* method according to any one of claims 1-4, wherein if at least one of 72T>C, 73A>G and 16356T>C genetic variants is detected, it is concluded that the subject comprises beneficial genetic variants protecting from suffering from a myocardial infarct.

6. The *in vitro* method according to any one of claims 1-5, wherein the method further comprises determining in the isolated sample one or more nuclear DNA genetic variants.

7. The *in vitro* method according to claim 6, wherein nuclear DNA genetic variants are selected from the group consisting of rs10507391, rs17222842, rs9315051, rs1333049, rs1746048, rs9982601, rs10455872, rs17465637, rs9818870, rs12526453, rs6725887, and combinations thereof.

8. The *in vitro* method according to any one of claims 1-7, wherein genetic variants are determined by amplification of SEQ ID NO: 6 or a fragment thereof, said fragment comprising the genetic variants 16145G>A, 16311T>C, 16356T>C 72T>C and 73A>G, with a set of primers selected from one or more of the following pairs of primers:
(a) Forward primer 5'-ATACACCAGTCTTGTAAACC-3' (SEQ ID NO: 1) and reverse primer 5'-TTGAGGAGGTAAGCTACATA-3' (SEQ ID NO: 2);
(b) Forward primers 5'-ATACACCAGTCTTGTAAACC-3' (SEQ ID NO: 1) and 5'-GAACTGTATCCGACATCTGG-3' (SEQ ID NO: 3) and reverse primers 5'-GGTTAATAGGGTGATAGACC-3' (SEQ ID NO: 4) and 5'-GGGGTTTGGTGGAAATTTTT-3' (SEQ ID NO: 5);
(c) Forward primer 5'-CACCCATCAACAACCGCTATG-3' (SEQ ID NO: 7) and reverse primer 5'-GGATGAGGCAGGAATCAAAGAC-3'(SEQ ID NO: 8).

9. The *in vitro* method according to any one of claims 1-8, further comprising considering one or more of the clinical parameters and/or features of the subject selected from hypercholesterolemia, hypertension, age, gender, systolic blood pressure, diastolic blood pressure, total-cholesterol levels, LDL-cholesterol levels, HDL-cholesterol, smoking habit, diabetes status, body mass index, triglyceride levels, hypolipemic drug consumption, blood pressure drug consumption, hypoglycaemic drug consumption and combinations thereof.

10. The *in vitro* method according to any one of claims 1-10, further comprising considering if the subject displays a clinical parameter selected from hypercholesterolemia, hypertension and combinations thereof.

11. A set of primers comprising as forward primer SEQ ID NO: 7 and as reverse primer SEQ ID NO: 8.

12. A method of deciding or recommending whether to initiate a prophylactic and/or therapeutic regimen of a subject suspected at risk of suffering from a cardiovascular disease, which method comprises:
a) detecting, *in vitro,* according to the method of claim 1, if one or more genetic variants are present in D-loop of mitochondrial DNA of SEQ ID NO: 6 from an isolated sample of a subject, wherein the variants are selected from one or more of 16145G>A, 16311T>C, 16356T>C, 72T>C and 73A>G and determining a risk score of suffering from a cardiovascular disease; and
b) if the subject is considered at risk of suffering from a cardiovascular disease, recommending a prophylactic and/or therapeutic regimen.
